# EUROPÄISCHE PATENTSCHRIFT

(11) **EP 2 168 533 B1**
(45) Veröffentlichungstag und Bekanntmachung des Hinweises auf die Patenterteilung: **23.02.2022**
(21) Anmeldenummer: 09010622.0
(22) Anmeldetag: 18.08.2009
(51) Int. Cl.: A61D 7/00

(54) **Spritze, insbesondere für veterinärmedizinische Anwendungen**
Syringe, particularly for veterinary applications
Seringue, notamment pour applications médicales vétérinaires

(30) Priorität: 26.09.2008 DE 102008049082
(43) Veröffentlichungstag der Anmeldung: 31.03.2010
(73) Patentinhaber: Henke-Sass, Wolf GmbH, 78532 Tuttlingen (DE)
(72) Erfinder: Rückert, Ralf, 78573 Wurmlingen (DE); Keuffel, Roland, 78579 Neuhausen ob Eck (DE)
(74) Vertreter: Patentanwälte Geyer, Fehners & Partner mbB

(56) Entgegenhaltungen:
- US-A- 3 543 603
- US-A- 3 827 601
- US-A- 5 951 516

## Beschreibung

Die vorliegende Erfindung betrifft eine Spritze, insbesondere für veterinärmedizinische Anwendungen, mit den Merkmalen des Oberbegriffs des Anspruches 1.

Solche Spritzen sind z.B. aus der US 5 951 516 A bekannt und werden z.B. im veterinärmedizinischen Bereich zum Injizieren von Medikamenten eingesetzt. Wichtig hierbei ist es sicherzustellen, daß dabei das Medikament nicht versehentlich Menschen injiziert wird.

Die US 3 543 603 A beschreibt einen Injektor mit einer Kanüle, bei dem über einen Dreh-Schiebe-Mechanismus oder einen Schiebe-Mechanismus eine federvorgespannte Kolbenstange ausgelöst wird, um ein Injizieren eines Fluids über die Kanüle zu bewirken.

Ausgehend davon ist es Aufgabe der Erfindung, die Spritze der eingangs genannten Art so weiterzubilden, daß ein unbeabsichtigtes Injizieren eines im Spritzenzylinder vorhandenen Fluids sicher vermieden werden kann.

Die Erfindung ist im Anspruch 1 definiert. Vorteilhafte Weiterbildungen sind in den abhängigen Ansprüchen angegeben.

Bei der erfindungsgemäßen Spritze wird somit, wenn die Schutzkappe in ihrer ersten Endstellung und das Sicherungselement in seiner ersten Sicherungsstellung steht, sichergestellt, daß die Kanüle nicht versehentlich eingestochen werden kann, da ihr vorderes Ende vollständig innerhalb der Schutzkappe liegt.

Bevorzugt ist das Schutzmodul so ausgebildet, daß in seiner Grundstellung die Schutzkappe in ihrer ersten Endstellung steht und das Sicherungselement sich in der ersten Sicherungsstellung befindet. Insbesondere können die erste Endstellung der Schutzkappe sowie die erste Sicherungsstellung des Sicherungselementes durch federnde Elemente so vorgespannt sein, daß ein Kraftaufwand nötig ist, um die Kanüle aus der ersten Endstellung und das Sicherungselement aus der ersten Sicherungsstellung zu bringen.

Selbst wenn das Sicherungselement in seiner zweiten Sicherungsstellung steht und die Kanüle versehentlich eingestochen wird, wird bei der erfindungsgemäßen Spritze immer noch ein Applizieren des im Spritzenzylinder befindlichen Fluids verhindert, da aufgrund des Schutzmoduls die Kolbenstange sowohl in der ersten als auch in der zweiten Sicherungsstellung des Sicherungselements gegen Verschiebungen arretiert ist. Erst wenn das Sicherungselement in seiner dritten Sicherungsstellung ist, kann die Kolbenstange bewegt werden. Es ist daher ein dreistufiger Sicherheitsmechanismus vorgesehen, der ein unbeabsichtigtes Applizieren eines Fluids aus dem Spritzenzylinder sicher verhindert.

Da die erfindungsgemäß Spritze so ausgebildet ist, daß das Sicherungselement von der zweiten Sicherungsstellung nur dann in die dritte Sicherungsstellung bringbar ist, wenn die Schutzkappe in ihrer zweiten Endstellung steht, kann dadurch die notwendige Einstichtiefe der Kanüle fest vorgegeben werden. Erst wenn diese Einstichtiefe erreicht ist, ist ein Applizieren des Fluids aus dem Spritzenzylinder möglich. Dadurch kann leicht sichergestellt werden, daß die Applizierung des Fluids, was insbesondere ein Medikament sein kann, auch die gewünschte Wirkung hat.

Das Sicherungselement kann als schwenkbar im Hauptkörper gelagerter Hebel ausgebildet sein. Damit ist es einfach möglich, ein Sicherungselement in drei verschiedene Sicherungsstellungen zu bringen.

So kann der Arretierabschnitt beispielsweise in der ersten und zweiten Sicherungsstellung zwei im Hauptkörper gelagerte Kugeln in eine Nut in der Kolbenstange drücken. Bei der Nut kann es sich insbesondere um eine radial umlaufende Nut handeln. Mit einem solchen Arretierabschnitt ist eine sichere Arretierung bzw. Blockierung der Kolbenstange gegen Verschiebungen möglich.

Der Arretierabschnitt kann im wesentlichen U-förmig mit zwei freien Schenkeln ausgebildet sein, wobei sich der Abstand der beiden Schenkel zum offenen Ende des U-förmigen Arretierabschnittes hin ändert. Der Abstand kann sich vergrößern oder vermindern und ist so gewählt, daß in der ersten und zweiten Sicherungsstellung die Kugeln sicher in die Nut gedrückt werden und in der dritten Sicherungsstellung der Abstand so groß ist, daß die Kugeln aus der Nut herausgedrückt werden können, so daß die Arretierung freigegeben wird.

Die Schutzkappe kann bei der erfindungsgemäßen Spritze einen Vorderabschnitt und einen Hauptabschnitt aufweisen, wobei der Vorderabschnitt lösbar mit dem Hauptabschnitt verbunden ist und ein Kanülenwerkzeug aufweist, mit dem die Kanüle vom Spritzenzylinder gelöst werden kann.

Bei dem Kanülenwerkzeug kann es sich beispielsweise um eine spezielle Ausbildung der vorderen Öffnung des Vorderabschnittes handeln. In diesem Fall kann die vordere Öffnung über die Kanüle bis zu dem Sockelabschnitt der Kanüle geschoben werden, wobei dann ein Formschluß zwischen der Öffnung und dem Sockelabschnitt vorliegt, so daß ein Verdrehen mittels des Vorderabschnittes möglich ist. Alternativ ist es möglich, an der Außenwandung des Vorderabschnittes ein für den Sockelabschnitt der Kanüle formschlüssiges Werkzeug auszubilden.

Die Schutzkappe weist insbesondere eine vordere Öffnung auf, durch die die Spitze der Kanüle bei der Bewegung der Schutzkappe von ihrer ersten in ihre zweite Endstellung hindurch läuft.

Die Spritze kann ferner eine Druckfeder zwischen der Kolbenstange und dem Hauptkörper aufweisen, wobei mittels der Druckfeder die Kolbenstange in ihre Ausgangsstellung gebracht ist, in der sie in der ersten und zweiten Sicherungsstellung des Sicherungselementes gegen Verschiebungen arretiert ist.

Ferner kann die Spritze einen Anschluß zum Befüllen aufweisen. Der Anschluß kann insbesondere mit einem Rückschlagventil ausgebildet sein, so daß die Rückwärtsbewegung des Kolbens nach dem Ausspritzen zu einem Befüllen des Spritzenzylinders über den Befüllungsanschluß führt. Der Befüllungsanschluß kann einen Flaschenhalter zum Aufnehmen eines Medikamentenbehälters oder auch einen Schlauchanschluß aufweisen.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Schnittansicht einer ersten Ausführungsform der erfindungsgemäßen Spritze;
- Fig. 2: eine Seitenansicht einer zweiten Ausführungsform der erfindungsgemäßen Spritze mit einer Teilschnittdarstellung des Stiftes 19;
- Fig. 3: eine Darstellung der Spritze gemäß Fig. 2, wobei der Sicherheitshebel in seiner zweiten Schwenkstellung steht;
- Fig. 4: eine Darstellung der Spritze gemäß Fig. 2, wobei die Spritze in eine Einspritzstelle eingestochen ist;
- Fig. 5: eine Ansicht der Spritze gemäß Fig. 2, wobei der Sicherheitshebel in seiner dritten Schwenkstellung steht;
- Fig. 6: eine Darstellung der Spritze gemäß Fig. 2, wobei der Sicherheitshebel in seiner dritten Sicherungsstellung steht und die Kolbenstange zum Ausspritzen des Mediums aus dem Spritzenzylinder zum Grundkörper 2 hinbewegt ist;
- Fig. 7a - 7c: perspektivische Teilansichten zur Erläuterung der Arretierung der Kolbenstange 11, und
- Fig. 8a - 8c: Schnittansichten von hinten zur Erläuterung der Arretierung der Kolbenstange 11.

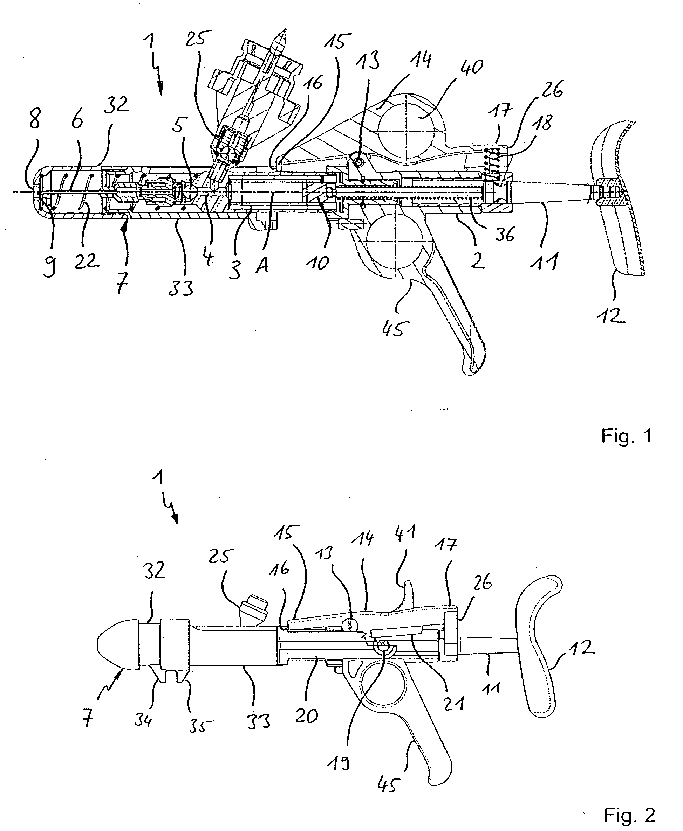

Bei der in Fig. 1 gezeigten Ausführungsformen umfaßt die erfindungsgemäße Spritze 1 einen Grundkörper 2, in dem ein Spritzenzylinder 3 angeordnet ist. Der Spritzenzylinder 3 ist ausspritzseitig über einen Kanal 4 und ein Rückschlagventil 5 mit einer Kanüle 6 verbunden.

Die Spritze 1 umfaßt ferner eine Schutzkappe 7 mit einer vorderen Öffnung 8, wobei die Schutzkappe 7 am Grundkörper 2 in axialer Richtung verschiebbar gelagert ist. In Fig. 1 ist die Schutzkappe 7 in ihrer ersten Endstellung, in der die Spitze 9 der Kanüle 6 nicht aus der Schutzkappe 7 nach vorne heraussteht.

In dem Spritzenzylinder 3 ist ein in Längsrichtung verschiebbarer Kolben 10 angeordnet, der mit einer im Grundkörper 2 geführten Kolbenstange 11 verbunden ist. Die Kolbenstange 11 steht nach hinten aus dem Grundkörper 2 heraus und ist mit einem Griffelement 12 verbunden. In Fig. 1 ist die Ausgangsstellung vom Kolben 10 und Kolbenstange 11 gezeigt.

Auf der Oberseite des Grundkörpers 2 ist ein um eine Achse 13 schwenkbarer Sicherheitshebel 14 angeordnet, der, wie nachfolgend noch im Detail beschrieben wird, in drei verschiedene Sicherungs- bzw. Schwenkstellungen gebracht werden kann.

Bei der Darstellung von Fig. 1 ist der Sicherheitshebel 4 in seiner ersten Schwenkstellung, in der sein vorderes Ende 15 an einen Anschlag 16 der Schutzkappe 7 anliegt, so daß die Schutzkappe 7 nicht zum Grundkörper 2 hin verschoben werden kann. Um den Sicherheitshebel 14 in seiner ersten Schwenkstellung zu halten, ist zwischen einem hinteren Ende 17 des Sicherheitshebels 14 und dem Grundkörper 2 eine Spiralfeder 18 angeordnet, die den Sicherheitshebel 14 in der ersten Schwenkstellung hält.

In diesem Zustand wird ein unbeabsichtigtes Einstechen der Kanüle 6 aufgrund der in der ersten Endstellung stehenden Schutzkappe 7 sicher verhindert.

Das gleiche gilt für die in Fig. 2 gezeigte Ausführungsform der erfindungsgemäßen Spritze 1. Die Ausführungsform von Fig. 2 unterscheidet sich von der von Fig. 1 nur durch die Ausbildung des Sicherheitshebels 14 und der Schutzkappe 7. Bei der Ausführungsform von Fig. 1 weist der Sicherheitshebel 14 eine Durchgangsbohrung 40 für den Zeigefinger des Bedieners auf. Der Sicherheitshebel 14 von Fig. 2 weist statt der Durchgangsbohrung 40 einen nach oben stehenden Fingeransatz 41 auf, der z.B. mit dem Zeigefinger von hinten umgriffen werden kann, um den Sicherheitshebel 14 aus seiner ersten Schwenkstellung zu bringen. Ansonsten sind die Sicherheitshebel 14 von Fig. 1 und 2 im wesentlichen identisch und weisen die gleiche Funktionalität auf.

Auch die Schutzkappe 7 von Fig. 1 und 2 sind im wesentlichen identisch und weisen die gleiche Funktionalität auf. Die Schutzkappe 7 von Fig. 2 weist im Vergleich zur Schutzkappe 7 von Fig. 1 noch Vorsprünge 34, 35 auf, deren Funktion später beschrieben wird.

Die Spritze 1 weist auf ihrer Unterseite ferner einen Fingergriff 45 auf.

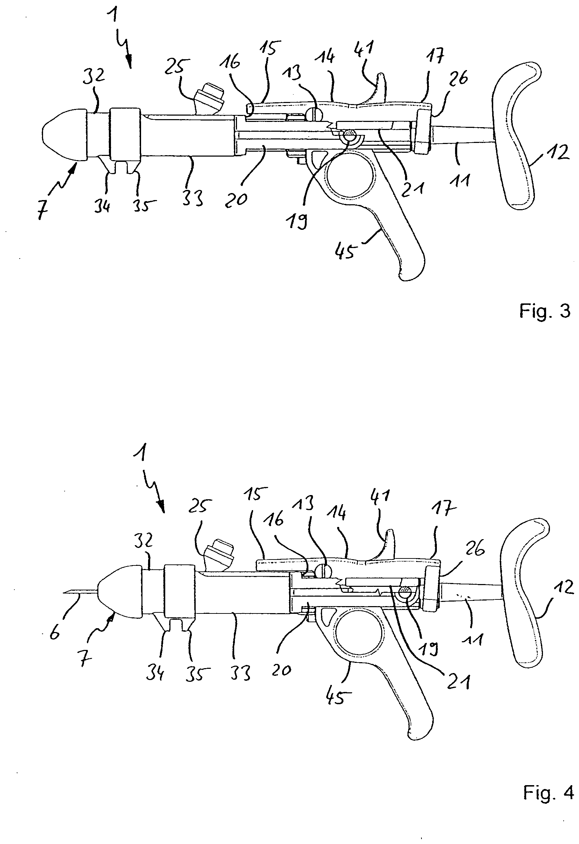

Bei Benutzung der Spritze 1 muß ein Bediener zunächst den Sicherheitshebel 14 von seiner ersten Schwenkstellung (Fig. 1, Fig. 2) in seine in Fig. 3 gezeigte zweite Schwenkstellung bringen (in Fig. 3 bis 6 ist die Ausführungsform der Spritze von Fig. 2 dargestellt). Dazu muß der Bediener lediglich mit dem Zeigefinger im hinteren Bereich des Sicherheitshebels 14 (also zwischen der Achse 13 und dem hinteren Ende 17) auf den Fingeransatz 41 des Sicherheitshebels 14 drücken, so daß das vordere Ende 15 und der Anschlag 16 außer Eingriff kommen, wie in Fig. 3 gezeigt ist. Der Sicherheitshebel 14, der nachfolgend auch Sicherungshebel 14 genannt wird, wird somit durch eine Schwenkbewegung von der ersten Sicherungsstellung in die zweite Schwenkstellung gebracht. Da diese Schwenkbewegung gegen die Federkraft der Spiralfeder 18 durchgeführt wird, muß der Bediener den Sicherheitshebel 14 aktiv in der zweiten Schwenkstellung halten.

Die zweite Schwenkstellung wird, wie in Fig. 3 gezeigt ist, durch einen senkrecht zur axialen Achse A der Spritze 1 verlaufenden Stift 19, der über ein Zwischenelement 20 mit der Schutzkappe 7 verbunden ist, festgelegt, an dem eine untere Anschlagschiene 21 des Sicherheitshebels 14 anliegt. Da die Anschlagschiene 21 in den Darstellungen von Fig. 2 - 6 hinter dem Zwischenelement 20 liegt und von diesem daher verdeckt wäre, ist in Fig. 2 - 6 der Stift 19 in einer Teilschnittdarstellung gezeigt. Auf der in Fig. 3 nicht sichtbaren Seite der Spritze 1 ist ebenfalls ein Zwischenelement 20 gehaltener Stift vorgesehen, der mit einer weiteren Anschlagschiene des Sicherheitshebels 14 zusammenwirkt.

Wenn der Bediener nun den Sicherheitshebel 14 in die in Fig. 3 gezeigte zweite Sicherungsstellung bringt und halt, kann er die Spritze 1 an der Einspritzstelle (z.B. bei einem Tier, hier nicht gezeigt) anlegen und nach vorne bewegen. In diesem Fall wird die Schutzkappe 7 an der durch die Einspritzstelle definierte Position bleiben, während der Grundkörper 2 relativ zur Schutzklappe 7 und damit auch die Kanüle 6 relativ zur Schutzkappe 7 bewegt und die Kanüle 6 dadurch in die Einspritzstelle hineingestochen wird. Die Schutzkappe 7 wird somit über die Kanüle 6 zurückgeschoben. Aufgrund einer zwischen dem Grundkörper 2 und der Schutzkappe 7 angeordneten Spiralfeder 22 ist dazu ein vorbestimmter Kraftaufwand notwendig.

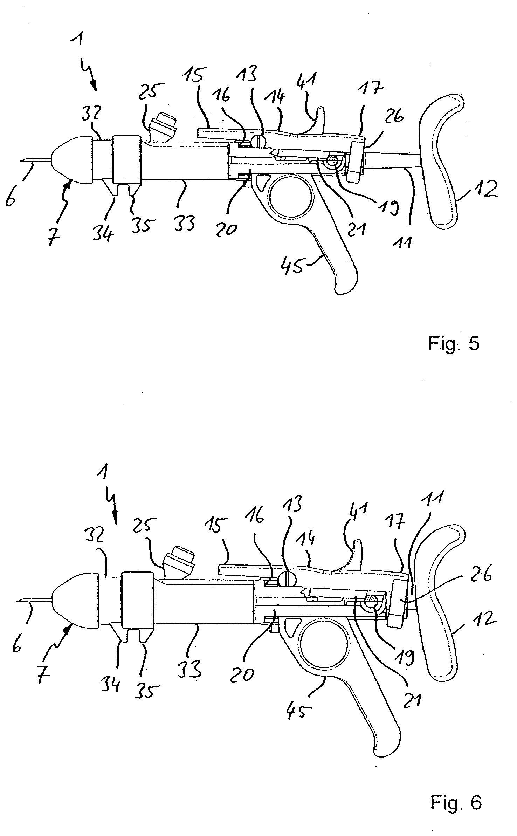

Bei dieser Relativbewegung zwischen Grundkörper 2 und Schutzkappe 7 gleitet das vordere Ende 15 des Sicherheitshebels 14 auf der Oberseite der Schutzkappe 7 sowie die Anschlagschiene 21 auf dem Stift 19, so daß der Sicherheitshebel 14 in seiner zweiten Sicherungsstellung bleibt und weder in die erste oder dritte Sicherungsstellung gebracht werden kann. Wenn der Stift 19 soweit relativ zur Anschlagschiene 21 verschoben ist, daß er hinter dem hinteren Ende der Anschlagschiene 21 liegt und somit die zweite Sicherungsstellung nicht mehr vorgibt (Fig. 4), kann der Bediener den Sicherheitshebel 14 von seiner zweiten Sicherungsstellung durch Drehen um die Achse 13 in seine in Fig. 5 gezeigte dritte Sicherungsstellung bringen.

Die dritte Sicherungsstellung 11 unterscheidet sich von der ersten und zweiten Sicherungsstellung dadurch, daß die Kolbenstange 11 nicht mehr arretiert ist, sondern in axialer Richtung bewegt werden kann. Somit kann der Bediener nun die Kolbenstange 11 in Richtung zum Grundkörper 2 hin bewegen (Fig. 6), wodurch der Kolben 10 im Spritzenzylinder 3 zum ausspritzseitigen Ende hin bewegt wird. Dadurch wird das sich im Spritzenzylinder 3 befindende Medikament über die Kanüle 6 in die Einspritzstelle eingebracht.

Somit kann das Medikament mit der erfindungsgemäßen Spritze 1 erst dann appliziert werden, wenn die Schutzkappe 7 in ihrer zweiten Endstellung steht und der Sicherheitshebel 14 in seiner dritten Sicherungsstellung steht. Dadurch wird eine versehentliche Applizierung des Medikamentes verhindert. Ferner wird durch die zweite Endstellung der Schutzkappe 7 die Einstichtiefe vorgegeben. Erst bei Erreichen der vorgegebenen Einstichtiefe kann das Medikament appliziert werden, wodurch das Einbringen des Medikamentes in der vorgegebenen Einstichtiefe gewährleistet werden kann.

Wenn die Spritze 1 nach Einbringen des Medikamentes aus der Einspritzstelle herausgezogen wird, gleitet die Schutzkappe 7 aufgrund der Spiralfeder 22 wieder in ihre erste Endstellung, wobei dabei auch gleichzeitig die Kolbenstange 11 aufgrund einer Kolbenfeder 36 und somit auch der Kolben 10 in seine Ausgangsstellung gebracht wird. Ferner wird der Sicherheitshebel 14 aufgrund der Spiralfeder 18 in seine erste Sicherungsstellung geschwenkt. Die Spritze 1 befindet sich somit wieder im in Fig. 1 bzw. 2 gezeigten Zustand, in dem ein versehentliches Stechen mit der Kanüle nicht möglich ist.

Aufgrund der Rückwärtsbewegung der Kolbenstange 11 wird in bekannter Weise über eine Zuführung 25 mit Rückschlagelement, die in den Kanal 6 mündet, die nächste zu applizierende Medikamentendosis in den Spritzenzylinder 3 gesaugt.

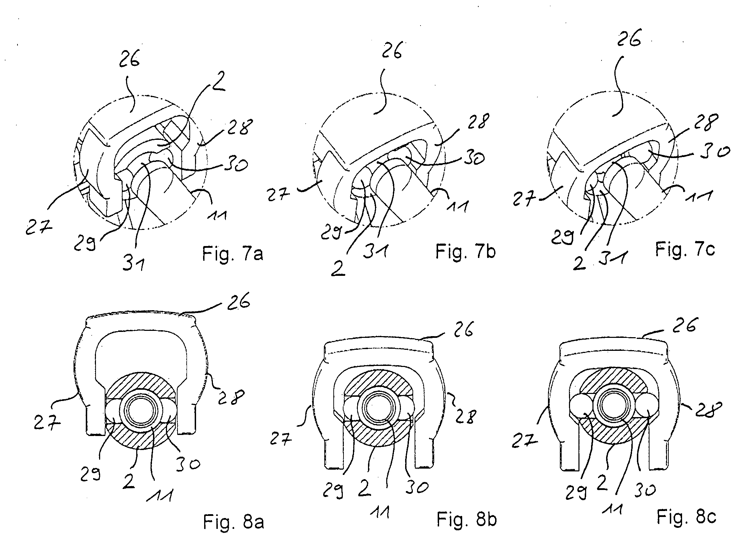

Die Arretierung der Kolbenstange 11 in der ersten und zweiten Sicherungsstellung sowie ihre Freigabe in der dritten Sicherungsstellung wird in Verbindung mit Fig. 7a - 7c und 8a - 8c beschrieben.

An dem hinteren Ende des Sicherungshebels 14 ist ein nach unten geöffnetes, U-förmiges Arretierelement 26 ausgebildet, dessen beide freien Schenkel 27, 28 im oberen Bereich einen größeren Abstand voneinander aufweisen als im unteren Bereich. Bei der in Fig. 7a und 8a gezeigten zweiten Sicherungsstellung drücken die beiden Schenkel 27 und 28 zwei in einem im Hauptkörper 2 ausgebildeten Kugelsitz angeordnete Kugeln 29, 30 in eine radial umlaufende Nut 31 in der Kolbenstange 11. Das gleiche gilt für die erste Sicherungsstellung (nicht gezeigt), in der auch der vordere Teil der beiden Schenkel 27, 28 die Kugeln 29, 30 in die Nut 31 drücken. Dadurch wird eine axiale Bewegung der Kolbenstange 11 blockiert. Ein unbeabsichtigtes Ausspritzen des Medikamentes aus dem Spritzenzylinder 3 wird somit wirksam verhindert.

Wenn der Sicherheitshebel 14 in seiner dritten Sicherungsstellung steht, sind die Kugeln 29 und 30 nicht mehr im engeren Bereich zwischen den beiden freien Schenkel 27 und 28 sondern im weiteren Bereich. Durch ein Schieben der Kolbenstange 11 ist es daher nun möglich, die beiden Kugeln 29 und 30 in radialer Richtung nach außen zu drücken, wie in Fig. 7c und 8c gezeigt ist, so daß die Kolbenstange 10 in axialer Richtung bewegbar ist. Die Arretierung ist somit aufgehoben.

Wenn der Sicherheitshebel 14 und somit das Arretierelement 26 aus der dritten Sicherungsstellung wieder in die zweite und die erste Sicherungsstellung gebracht wird, werden die beiden Kugeln 29 und 30 aufgrund der freien Schenkel 27 und 28, deren Abstand zum offenen Ende hin abnimmt, wieder in die umlaufende Nut 31 in der Kolbenstange 10 gedrückt (dies ist natürlich nur möglich, wenn die Kolbenstange 11 wieder in ihrer Ausgangsstellung steht), wodurch die Kolbenstange 10 wieder gegen Bewegungen in axialer Richtung arretiert ist.

Die hier beschriebene Schutzkappe 7 weist einen Vorderabschnitt 32 auf, der auf einen hinteren Kappenabschnitt 33 aufgeschraubt ist. Somit kann nach Abschrauben des Vorderabschnitts 32 die Kanüle 6 gewechselt werden, die ihrerseits lösbar mit dem Spritzenzylinder 3 verbunden ist (z.B. verschraubt).

Bei der Ausführungsform von Fig. 2 - 6 weist der Vorderabschnitt die zwei Vorsprünge 34, 35 auf, die so in ihrer Form und Abmessung gewählt sind, daß im abgeschraubten Zustand des Vorderabschnittes 32 mit den Vorsprüngen 34 und 35 die Kanüle 6 abgeschraubt werden kann. Damit kann ein direkter Kontakt der Kanüle mit der Haut des Benutzers vermieden werden. Natürlich können die Vorsprünge 34, 35 auch zum Aufschrauben einer neuen Kanüle verwendet werden.

Bei der in Fig. 1 gezeigten Ausführungsform der Spritze 1 ist die vordere Öffnung 8 so ausgebildet, daß der abgeschraubte Vorderabschnitt 32 herumgedreht und mit der vorderen Öffnung 8 zuerst über die Kanüle 6 und über den Kanülenansatz geschoben werden kann, wobei zwischen der Öffnung 8 und dem Kanülenansatz Formschluß vorliegt. Dadurch kann der Vorderabschnitt 32 wiederum als Werkzeug zum Lösen und/oder Befestigen der Kanüle 6 genutzt werden.

## Patentansprüche

1. Spritze, insbesondere für veterinärmedizinische Anwendungen, mit einem Grundkörper (2), der einen Spritzenzylinder (3) aufweist, dessen ausspritzseitiges Ende mit einer Kanüle (6) verbunden ist und in dem ein mit einer Kolbenstange (11) verbundener Kolben (10) verschiebbar angeordnet ist,
wobei die Spritze ein Schutzmodul aufweist, das
eine am Grundkörper (2) verschiebbar gelagerte Schutzkappe (7), die in ihrer ersten Endstellung die Kanüle (6) so aufnimmt, dass sie mit ihrem vorderen Ende (9) nicht aus der Schutzkappe (7) heraussteht, und
ein Sicherungselement (14) aufweist, das von einer ersten Sicherungsstellung in eine zweite Sicherungsstellung und danach in eine dritte Sicherungsstellung bringbar ist,
wobei das Sicherungselement (14) in seiner ersten Sicherungsstellung die Schutzkappe (7) in ihrer ersten Endstellung arretiert,
die Schutzkappe (7) in der zweiten Sicherungsstellung des Sicherungselementes (14) zum Grundkörper (2) hin in ihre zweite Endstellung, in der das vordere Ende (9) der Kanüle (6) aus der Schutzkappe (7) heraussteht, verschiebbar ist,
und wobei mittels der Kolbenstange (11) der Kolben (10) im Spritzenzylinder (3) in Richtung zum ausspritzseitigen Ende hin bewegt werden kann, wenn das Sicherungselement (14) in seiner dritten Sicherungsstellung ist, in die es aus der zweiten Sicherungsstellung nur bringbar ist, wenn die Schutzkappe (7) in ihrer zweiten Endstellung steht,
**dadurch gekennzeichnet, dass**
das Sicherungselement (14) in seiner dritten Sicherungsstellung eine in der ersten und zweiten Sicherungsstellung durch das Schutzmodul bedingte Arretierung der Kolbenstange (11) gegenüber Verschiebungen freigibt und
das Sicherungselement (14) einen Arretierabschnitt (26) aufweist, der in der ersten und zweiten Sicherungsstellung die Arretierung der Kolbenstange (11) bewirkt.

2. Spritze nach Anspruch 1, bei der das Sicherungselement (14) als schwenkbar am Hauptkörper (2) gelagerter Hebel ausgebildet ist.

3. Spritze nach Anspruch 1 oder 2, bei der die Schutzkappe (7) in ihrer ersten Endstellung federnd gehalten ist.

4. Spritze nach einem der obigen Ansprüche, bei der das Sicherungselement (10) federnd in der ersten Sicherungsstellung gehalten ist.

5. Spritze nach einem der obigen Ansprüche, bei der der Arretierabschnitt (26) in der ersten und zweiten Sicherungsstellung zwei im Hauptkörper (2) gelagerte Kugeln (29, 30) in eine Nut in der Kolbenstange (11) drückt.

6. Spritze nach einem der obigen Ansprüche, bei der der Arretierabschnitt (26) im wesentlichen U-förmig mit zwei freien Schenkeln (27, 28) ausgebildet ist, wobei sich der Abstand der beiden Schenkel (27, 28) zum offenen Ende des U-förmigen Arretierabschnittes (26) hin ändert.

7. Spritze nach einem der obigen Ansprüche, bei der die Schutzkappe (7) einen Vorderabschnitt (32) und einen Hauptabschnitt (33) aufweist, wobei der Vorderabschnitt (32) lösbar mit dem Hauptabschnitt (33) verbunden ist und ein Kanülenwerkzeug (34, 35) aufweist, mit dem die Kanüle vom Spritzenzylinder (3) gelöst werden kann.

## Claims

1. A syringe, in particular for veterinary-medicine applications, having a main body (2), which has a syringe cylinder (3), whose discharge-side end is connected to a cannula (6) and in which a piston (10), which is connected to a piston rod (11), is situated so it is displaceable,
wherein the syringe comprises a protective module, which has
a protective cap (7), mounted so it is displaceable on the main body (2), which receives the cannula (6) in its first terminal position so that the front end (9) thereof does not protrude from the protective cap (7), and
a securing element (14), which can be moved from a first securing position into a second securing position and then into a third securing position,
the securing element (14), in its first securing position, locking the protective cap (7) in its first terminal position,
the protective cap (7) being displaceable toward the main body (2) into its second terminal position, in which the front end (9) of the cannula (6) protrudes from the protective cap (7), if the securing element (14) is in the second securing position,
and wherein the piston (10) can be moved in the syringe cylinder (3) in the direction toward the discharge-side end using the piston rod (11) if the securing element (14) is in its third securing position, into which it is only movable from the second securing position when the protective cap (7) is in its second terminal position,
**characterized in that**
the securing element (14), in its third securing position, releases a locking of the piston rod (11) relative to displacements, which is caused in the first and second securing positions by the protective module, and
the securing element (14) has a locking section (26), which causes the locking of the piston rod (11) in the first and second securing positions.

2. The syringe according to Claim 1, wherein the securing element (14) is implemented as a lever mounted so it is pivotable on the main body (2).

3. The syringe according to Claim 1 or 2, wherein the protective cap (7) is spring-loaded in its first terminal position.

4. The syringe according to one of the preceding claims, wherein the securing element (10) is spring-loaded in the first securing position.

5. The syringe according to one of the preceding claims, wherein the locking section (26) presses two balls (29, 30), which are mounted in the main body (2), into a groove in the piston rod (11) in the first and second securing positions.

6. The syringe according to one of the preceding claims, wherein the locking section (26) is implemented as essentially U-shaped having two free legs (27, 28), the spacing of the two legs (27, 28) changing toward the open end of the U-shaped locking section (26).

7. The syringe according to one of the preceding claims, wherein the protective cap (7) has a front section (32) and a main section (33), the front section (32) being removably connected to the main section (33) and having a cannula tool (34, 35), using which the cannula can be removed from the syringe cylinder (3).

## Revendications

1. Seringue, destinée en particulier à des applications vétérinaires, ladite seringue comprenant un corps de base (2) qui comporte un cylindre de seringue (3) dont l'extrémité côté injection est reliée à une canule (6) et dans lequel un piston (10) relié à une tige de piston (11) est disposé de manière coulissante,
la seringue comportant un module de protection qui comporte
un capuchon de protection (7) qui est monté de manière coulissante sur le corps de base (2) et qui reçoit, dans sa première position extrême, la canule (6) de sorte que son extrémité avant (9) ne fasse pas saillie du capuchon de protection (7), et
un élément de sécurité (14) qui peut être amené d'une première position de sécurité dans une deuxième position de sécurité puis dans une troisième position de sécurité,
l'élément de sécurité (14) bloquant dans sa première position de sécurité le capuchon de protection (7) dans sa première position d'extrémité,
le capuchon de protection (7) pouvant coulisser, lorsque l'élément de sécurité (14) est dans la deuxième position de sécurité, en direction du corps de base (2) jusque dans sa deuxième position d'extrémité dans laquelle l'extrémité avant (9) de la canule (6) fait saillie du capuchon de protection (7),
et le piston (10) pouvant être déplacé dans le cylindre de seringue (3) en direction de l'extrémité côté injection au moyen de la tige de piston (11) lorsque l'élément de sécurité (14) est dans sa troisième position de sécurité dans laquelle il ne peut quitter la deuxième position de sécurité que si le capuchon de protection (7) est dans sa deuxième position d'extrémité,
**caractérisé en ce que**
l'élément de sécurité (14) libérant dans sa troisième position de sécurité un blocage anti-coulissement de la tige de piston (11) réalisé par le module de protection dans les première et deuxième positions de sécurité, et l'élément de sécurité (14) comportant une portion de blocage (26) qui réalise le blocage de la tige de piston (11) dans les première et deuxième positions de blocage.

2. Seringue selon la revendication 1, dans laquelle l'élément de sécurité (14) est conçu sous la forme d'un levier monté de manière pivotante sur le corps principal (2).

3. Seringue selon la revendication 1 ou 2, dans laquelle le capuchon de protection (7) est maintenu élastiquement dans sa première position d'extrémité.

4. Seringue selon l'une des revendications précédentes, dans laquelle l'élément de sécurité (10) est maintenu élastiquement dans la première position de sécurité.

5. Seringue selon l'une des revendications précédentes, dans laquelle la portion de blocage (26) presse, dans la première et la deuxième position de sécurité, deux billes (29, 30), montées dans le corps principal (2), dans une rainure ménagée dans la tige de piston (1.1).

6. Seringue selon l'une des revendications précédentes, dans laquelle la portion de blocage (26) est sensiblement en forme de U comportant deux branches libres (27, 28), la distance entre les deux branches (27, 28) variant en direction de l'extrémité ouverte de la portion de blocage en forme de U (26).

7. Seringue selon l'une des revendications précédentes, dans laquelle le capuchon de protection (7) comporte une portion avant (32) et une portion principale (33), la portion avant (32) étant reliée de manière amovible à la portion principale (33) et comportant un outil de canule (34, 35) au moyen duquel la canule peut être détachée du corps de seringue (3).
